# FASCICULE DE BREVET EUROPEEN

(11) **EP 0 655 232 B1**
(45) Date de publication et mention de la délivrance du brevet: **23.06.1999**
(21) Numéro de dépôt: 94402409.0
(22) Date de dépôt: 26.10.1994
(51) Int. Cl.: A61F 5/01, A61F 5/04

(54) **Orthèse cervicale pour intervention d'urgence**
Cervicalorthese für den Notfall
Emergency cervical orthosis

(30) Priorité: 26.10.1993 FR 9312735
(43) Date de publication de la demande: 31.05.1995
(73) Titulaire: Biron, Stéphane, F-85000 La Roche-sur-Yon (FR)
(72) Inventeur: Biron, Stéphane, F-85000 La Roche-sur-Yon (FR)
(74) Mandataire: Jolly, Jean-Pierre

(56) Documents cités:
- EP-A- 0 121 718
- DE-C- 811 256
- GB-A- 1 544 874
- US-A- 2 980 110
- US-A- 4 232 663
- US-A- 4 325 363

## Description

La présente invention concerne une orthèse cervicale, du type des colliers cervicaux.

L'orthèse cervicale conforme à l'invention est plus particulièrement destinée à être utilisée dans des conditions d'urgence, notamment sur des blessés par accident, qui doivent être transportés par ambulance vers des hôpitaux. C'est à cette utilisation que l'on se réfèrera plus particulièrement ci-après, mais l'orthèse pourra également être utilisée dans toute application des orthèses de la technique antérieure.

On sait que les colliers cervicaux d'un type connu sont habituellement réalisés en un matériau rigide, par exemple en une matière plastique, en une ou plusieurs parties articulées entre elles, qui épousent la forme du cou et du menton du patient et qui assurent une immobilisation complète de la tête et du cou par rapport au tronc, tout en étant radiotransparentes, afin de permettre des radiographies du blessé.

Ces colliers cervicaux présentent divers inconvénients :
- d'une part, ils sont relativement complexes, donc coûteux ;
- d'autre part, ils doivent être réalisés en plusieurs dimensions, de manière à pouvoir équiper des patients de tailles différentes ;
- enfin, ils sont d'une utilisation compliquée, car il faut généralement avoir recours à deux personnes pour les mettre en place.

On connaît aussi des dispositifs métalliques, comprenant généralement des barrettes en aluminium ou en un alliage d'aluminium, que l'on met en forme pour les adapter à la morphologie des patients. Ces dispositifs présentent les mêmes inconvénients que les précédentes orthèses. Ils sont de plus difficilement adaptables, cassants à la longue et non radiotransparents.

On connaît, pour des malaises bénins tels que des torticolis, des petits colliers cervicaux constitués par une bande d'une matière plastique expansée gainée d'un tricot, que l'on enroule autour du cou du patient. Ces colliers sont simples et peu coûteux, mais ne conviennent qu'à une catégorie limitée d'utilisations, car ils n'assurent qu'une immobilisation très relative.

On connaît enfin, par US-A-2 980 110, une orthèse cervicale comprenant une bande dans laquelle des fentes latérales déterminent des languettes, qui peuvent supporter la tête du patient et prendre appui sur ses épaules. Cette bande est en un plastique lourd rigide permettant le doublage éventuel par rivetage à l'aide d'une bande similaire. Cette orthèse comporte donc un rembourrage formant coussin en un tissu de jersey, apte à éviter de blesser le patient.

La présente invention vise à remédier aux inconvénients des colliers cervicaux de la technique antérieure en proposant un collier simple, peu coûteux et facile à mettre en place, qui puisse être substitué avec avantage aux différents colliers mentionnés ci-dessus.

L'invention vise également à proposer un collier de ce type qui puisse être fabriqué de façon simple à partir de matériaux aisément disponibles.

A cet effet, l'invention a pour objet une orthèse cervicale d'urgence du type des colliers cervicaux, comprenant une première bande comportant une partie centrale semi-rigide sensiblement continue, à laquelle sont attenantes de chaque côté des languettes souples, qui s'étendent depuis cette partie centrale jusqu'aux bords de la bande, cette dernière étant destinée, par sa partie centrale, à enserrer, en le maintenant, le cou d'un patient, éventuellement avec recouvrement au moins partiel de ses extrémités, tandis que les languettes attenantes épousent en se déformant le profil de raccordement du cou au tronc et à la tête du patient, cette orthèse étant caractérisée en ce que la première bande est en une mousse de matière plastique et présente une face plane et une face à faible convexité, l'épaisseur de cette première bande décroissant depuis sa partie centrale jusqu'aux extrémités des languettes, et en ce qu'une seconde bande en un matériau plus souple est disposée dans le prolongement de la première bande et est attenante à une extrémité de celle-ci, de manière à pouvoir être enroulée autour d'elle.

Les languettes peuvent être séparées les unes des autres par de simples entailles pratiquées dans la première bande de mousse de matière plastique, perpendiculairement à la direction longitudinale de la bande.

Comme indiqué ci-dessus, afin de présenter une rigidité suffisante dans sa partie centrale et une plus grande souplesse au niveau des languettes, la première bande a une épaisseur qui décroît à partir de sa partie centrale jusqu'aux extrémités des languettes.

L'extrémité libre de la seconde bande pourra être rendue solidaire par des moyens appropriés de la première bande (ou d'elle-même, en cas de recouvrement).

Il est clair que l'orthèse cervicale conforme à l'invention pourra être fabriquée de façon extrêmement simple en découpant de façon appropriée une bande de mousse de matière plastique.

On notera que, comme les orthèses cervicales de la technique antérieure, les orthèses conformes a l'invention sont transparentes aux rayons X et se prêtent donc à des radiographies du patient qui les porte.

Les dessins annexés représentent, à titre d'exemple non limitatif, une forme de réalisation de l'orthèse conforme à l'invention. Sur ces dessins :

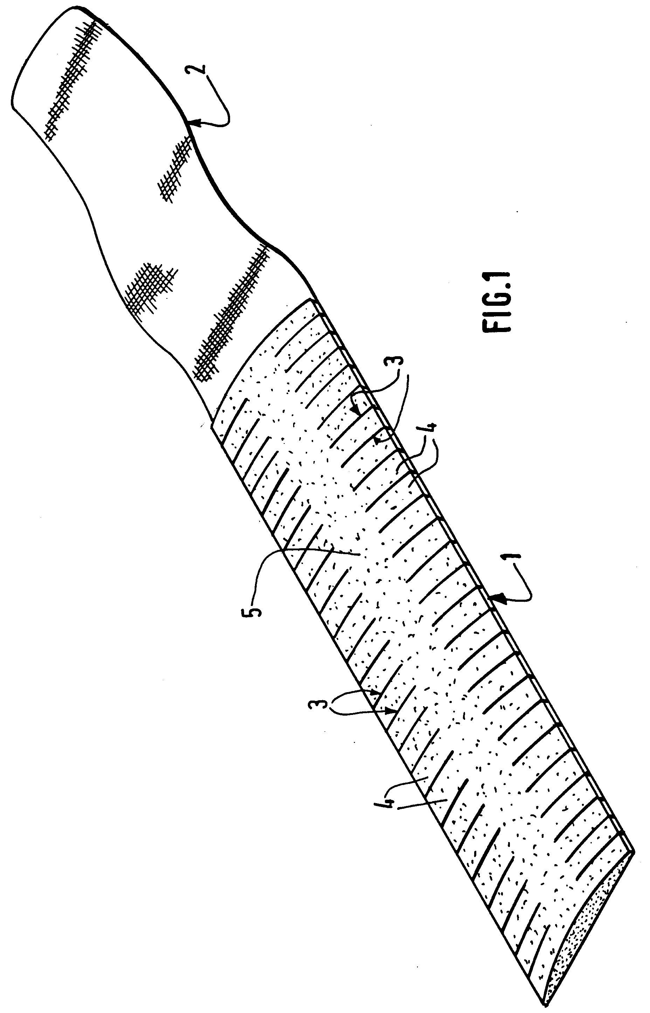

La figure 1 est une vue en perspective de l'orthèse à plat, en position de conditionnement et de stockage ;
La figure 2 est une vue en perspective de l'orthèse en position d'utilisation.

Cette orthèse comprend essentiellement une première bande 1 en une matière plastique semi-rigide telle qu'une mousse de matière plastique et, dans la forme de réalisation représentée sur les dessins, une seconde bande 2, en une matière plus souple, fixée à une extrémité de la bande 1 et prolongeant celle-ci.

La bande 1 est découpée latéralement, à partir de chacun de ses bords, par des entailles 3, perpendiculaires à la direction longitudinale de la bande, qui définissent des languettes 4 de chaque côté d'une bande centrale non découpée 5. Ces languettes 4 pourraient naturellement avoir une forme différente de celle représentée sur les dessins.

Bien entendu, l'orthèse pourra être réalisée en différentes tailles, mais, en général, deux tailles suffiront, une taille adulte et une taille enfant, la mise à longueur de l'orthèse s'effectuant par simple découpe sur les lieux de l'utilisation.

Un avantage majeur de l'orthèse conforme à l'invention est donc qu'elle s'applique à un large éventail de patients, avec une taille déterminée, car les languettes épousent les courbures anatomiques. Par ailleurs, il n'y a pas de parties dures saillantes pouvant blesser le patient. Les découpes peuvent permettre, éventuellement, d'adapter la longueur mais, surtout, de dégager une plaie ou de permettre un évidement trachéal.

Un autre avantage extrêmement important du collier conforme à l'invention est qu'il est monobloc et qu'il peut être conditionné à plat, ce qui facilite aussi bien son stockage que sa manipulation et sa mise en place sur le patient.

La partie centrale 5 de la bande a une épaisseur supérieure à celle des languettes 4 attenantes, de manière que la partie 5 soit relativement rigide, alors que les languettes 4 ont une plus grande souplesse permettant de les déformer. Dans une forme de réalisation simple représentée sur les dessins, l'épaisseur de la bande 1 décroît depuis la portion centrale de la partie 5 jusqu'à chacun des bords latéraux de la bande. L'une des faces de la bande 1 est donc plane, tandis que sa face opposée est légèrement convexe.

La bande 1 peut être, par exemple, en un matériau thermoformable de forte dénsité tel que le TEPEFORM, le PODOFORM ou le MULTIFORM (marques déposées), tandis que la bande 2, nettement plus souple, peut elle aussi être réalisée en un matériau thermoformable, mais de densité beaucoup plus faible, tel que le PLATAZOTE (marque déposée).

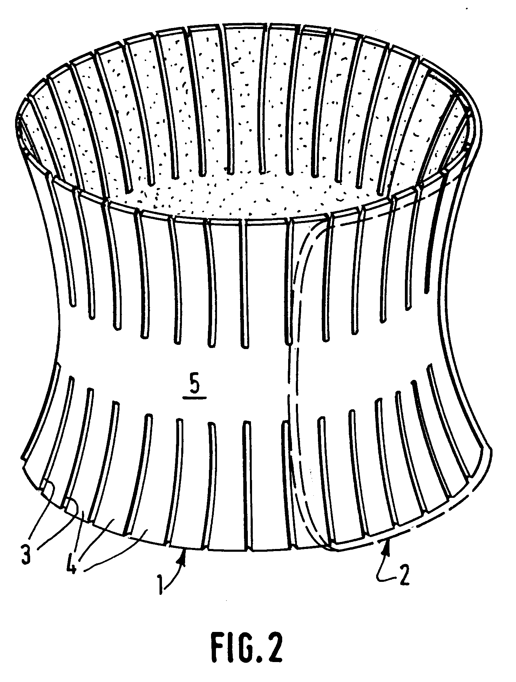

Comme représenté sur la figure 2, l'orthèse conforme à l'invention est enroulée autour du cou du patient, avec la face convexe de la bande 1 au contact du cou. Les deux extrémités de la bande 1 peuvent être contiguës ou il peut y avoir un recouvrement partiel entre elles. La bande 2, représentée en tiretés sur la figure 2, est ensuite enroulée à l'extérieur de la bande 1 pour serrer fortement celle-ci contre le cou du patient. Un simple ruban adhésif suffit pour rendre l'extrémité de cette bande 2 solidaire de la bande 1 ou d'elle-même, si elle fait un tour complet autour du cou du patient.

Lorsque le collier est mis en place autour du cou d'un patient, la partie centrale 5 de la bande 1 vient s'appliquer contre le cou, tandis que les languettes 4 s'écartent les unes des autres, à la manière de pétales de fleur, pour épouser la morphologie du patient, en prenant appui sur son sternum, sur ses épaules et sur le haut du dos, par les languettes disposées en partie basse, et en constituant un support mentonnier et occipital, par les languettes supérieures. La bande souple 2 enroulée autour de la bande 1 et rendue solidaire de celle-ci ou fixée sur elle-même assure la rigidité et le maintien de l'ensemble.

L'orthèse conforme à l'invention se prête donc particulièrement bien à une utilisation par des équipes de premier secours en cas d'accident de la route, en vue d'assurer le maintien du rachis cervical, dès qu'une lésion de la tête ou à la moelle épinière d'un blessé est à redouter.

Elle peut être mise en place par une unique personne et est prête à l'emploi dans les véhicules de secours.

Son coût est particulièrement faible et elle est lavable, désinfectable, et, éventuellement, réutilisable.

## Revendications

1. Orthèse cervicale d'urgence du type des colliers cervicaux, comprenant une première bande (1) comportant une partie centrale semi-rigide (5) sensiblement continue, à laquelle sont attenantes de chaque côté des languettes souples (4), qui s'étendent depuis cette partie centrale (5) jusqu'aux bords de la bande, cette dernière étant destinée, par sa partie centrale (5), à enserrer, en le maintenant, le cou d'un patient, éventuellement avec recouvrement au moins partiel de ses extrémités, tandis que les languettes attenantes (4) épousent en se déformant le profil de raccordement du cou au tronc et à la tête du patient, cette orthèse étant caractérisée en ce que la première bande (1) est en une mousse de matière plastique et présente une face plane et une face à faible convexité, l'épaisseur de cette première bande (1) décroissant depuis sa partie centrale (5) jusqu'aux extrémités des languettes (4), et en ce qu'une seconde bande (2) en un matériau plus souple est disposée dans le prolongement de la première bande (1) et est attenante à une extrémité de celle-ci, de manière à pouvoir être enroulée autour d'elle.

## Claims

1. An emergency cervical orthosis of the cervical collar type, comprising a first band (1) having a substantially continuous semi-rigid central part (5) with which flexible tongues (4) are contiguous on either side, which tongues extend from this central part (5) up to the edges of the band, the latter being intended to enclose, while supporting it, with its central part (5), the neck of a patient, optionally with at least partial overlapping of its ends, whereas the contiguous tongues (4), by deforming, adapt to the profile connecting the neck to the body and to the head of the patient, said orthosis being characterised in that the first band (1) is made of a foam of plastics material and has one flat surface and one slightly convex surface, the thickness of this first band (1) decreasing from its central part (5) up to the ends of the tongues (4), and in that a second band (2) of a more flexible material is provided in extension of the first band (1) and adjoins one end thereof in such a way that it can be wrapped around it.

## Patentansprüche

1. Zervikalorthese für Notfälle in der Art einer Zervikalmanschette, welche einen ersten Streifen (1) mit einem im wesentlichen durchgehenden halbsteifen Mittelbereich (5) aufweist, an welchem zu beiden Seiten elastische Zungen (4) angesetzt sind, die sich von diesem Mittelbereich (5) bis zu den Kanten des Streifens erstrecken, wobei letzterer dazu bestimmt ist, mit seinem Mittelbereich (5) den Hals eines Patienten zu umschließen und aufrecht zu halten, gegebenenfalls mit zumindest teilweiser Überdeckung seiner Enden, wohingegen die angesetzten Zungen (4) sich unter Verformung an den Umriß im Anschlußbereich des Halses an den Körper und dem Kopf des Patienten anlegen, **dadurch gekennzeichnet**, daß der erste Streifen (1) aus einem Kunststoffschaum besteht und eine ebene Fläche sowie eine leicht konvexe Fläche aufweist, wobei die Dicke dieses ersten Streifens (1) von seinem Mittelbereich (5) bis zu den Enden der Zungen (4) abnimmt, und daß ein zweiter Streifen (2) aus einem Werkstoff mit höherer Elastizität in der Verlängerung des ersten Streifens (1) angeordnet und an einem Ende desselben in der Weise angesetzt ist, daß er um den ersten Streifen wickelbar ist.
